# FASCICULE DE BREVET EUROPEEN

(11) **EP 1 136 572 B1**
(45) Date de publication et mention de la délivrance du brevet: **07.07.2004**
(21) Numéro de dépôt: 01400720.7
(22) Date de dépôt: 20.03.2001
(51) Int. Cl.: G01N 33/12, G01N 33/68, C12Q 1/68

(54) **Procédé et installation pour confirmer l'absence dans la viande de protéines prion infectieuses**
Verfahren und Vorrichtung zur Bestätigung der Abwesenheit von infektiösen Prion Proteinen in Fleisch
Method and device for confirming the absence of infectious prion proteins in meat

(30) Priorité: 24.03.2000 FR 0003775
(43) Date de publication de la demande: 26.09.2001
(73) Titulaire: Eurofins Scientific, 44300 Nantes (FR)
(72) Inventeur: Martin, Gilles, 90427 Nürnberg (DE)
(74) Mandataire: Laget, Jean-Loup

(56) Documents cités:
- WO-A-97/37227
- DE-C- 19 756 989
- FR-A- 2 762 842
- FR-A- 2 779 261
- GB-A- 2 246 500
- Annales de Gembloux, vol 103, pages 52-57, 1997. Portetelle d et al. " Meat traceability" XP002158736
- Dansk Veterinaertidsskrift, vol 80, pages 196-197. Petersen Lars Erik, " Meat control from stall to table." XP002158737
- Acta Neuropathologica, vol 98, Novembre 1999, pages 437-443; Schaller O et al. " Validation of a Western immunoblotting procedure for bovine PrPSc detection and its use as a rapid surveillance method for the diagnosis of bovine spongiform encephalopathy." XP002158738
- Computers and electronics in Agriculture; vol 24, Novembre 1999, pages 99-108. Wismans wW; " Identification and registration of animals in the European Union". XP002158739

## Description

La présente invention concerne un procédé pour confirmer l'absence de protéines prion (PrPsc ou PrPres) infectieuses spécifiques d'encéphalopathies spongiformes transmissibles (EST), telles que l'encéphalopathie spongiforme bovine ou la maladie de scrapie ou la chronic wasting disease, dans des pièces de viande, autres que le cerveau ou la moelle épinière, issues notamment de bovins ou de moutons, et en cours de commercialisation ainsi qu'une installation pour la mise en oeuvre du procédé.

Les maladies du prion ou encéphalopathies spongiformes transmissibles sont un groupe de maladies neurodégénératives qui affectent à la fois les humains et les animaux et qui peuvent être transmises entre mammifères en particulier par ingestion de tissus infectés. Ces maladies sont dues à l'altération de la configuration moléculaire d'une protéine prion PrP par un mécanisme inconnu qui transforme cette protéine en particules infectieuses appelées protéines prion PrPsc ou PrPres. Ces protéines infectieuses peuvent être distinguées des protéines normales par leur insolubilité, leur résistance partielle aux protéases et identifiées par différents anticorps (kits immunologiques ELISA, Western Blot, etc.).

Le fait que la maladie de Kreutzfeld Jacob chez l'humain puisse être initiée par l'ingestion de tissus d'animaux infectés d'encéphalopathie spongiforme transmissible génère une méfiance importante du consommateur à l'encontre de la consommation de viande. Il est donc devenu impératif, pour des raisons économiques et de santé publique, de mettre au point des procédés permettant de rassurer le consommateur et de certifier à ce dernier l'absence de protéines infectieuses dans la viande consommée.

WO 97/37227 D1, qui est considéré comme l'état de la technique le plus proche, décrit un procédé pour confirmer l'absence de protéines prion (PrPsc) infectieuses spécifiques d'encéphalopathies spongiformes transmissibles (EST), telles que l'encéphalopathie spongiforme bovine ou la maladie de scrapie ou le chronic wasting disease, dans des pièces de viande, autres que le cerveau ou la moelle épinière, issues notamment de bovins, de moutons ou de gibier, et en cours de commercialisation avec un test d'identification des protéines.

Toutefois, la difficulté réside aujourd'hui dans le fait que les pièces de viande commercialisées ne sont pas à ce jour directement analysables pour détecter la présence ou l'absence de protéines prion PrPsc infectieuses spécifiques d'encéphalopathies spongiformes transmissibles. En effet, les tests commercialisés ne permettent de détecter la présence de protéines prion infectieuses que dans le système nerveux central, en particulier le cerveau ou la moelle épinière, la sensibilité de ces tests étant insuffisante pour l'appliquer à d'autres parties de l'animal. On sait par ailleurs que les méthodes de traçabilité mises au point, qui consistent à indiquer sur chaque pièce de viande l'origine de cette pièce de viande et en particulier l'individu dont elle est issue, ne sont pas exemptes de fraude et sont difficiles à mettre en oeuvre en pratique pour les produits transformés.

Un but de la présente invention est de proposer un procédé qui permet, de par sa mise en oeuvre, de garantir de manière certaine l'absence de protéines prion infectieuses dans la pièce de viande analysée, cette pièce de viande pouvant être un morceau quelconque de l'animal.

Un autre but de la présente invention est de mettre au point un procédé du type précité qui permet de garantir un tel résultat indépendamment de la transformation à laquelle la viande a été soumise.

Un autre but de la présente invention est de proposer un procédé du type précité qui permet d'obtenir un tel résultat en l'absence de toute traçabilité et donc de tout marquage du produit final.

A cet effet, l'invention a pour objet un procédé pour confirmer l'absence de protéines prion (PrPsc) infectieuses spécifiques d'encéphalopathies spongiformes transmissibles (EST), telles que l'encéphalopathie spongiforme bovine ou la maladie de scrapie ou le chronic wasting disease, dans des pièces de viande, autres que le cerveau ou la moelle épinière, issues notamment de bovins, de moutons ou de gibier et en cours de commercialisation, caractérisé en ce qu'on pratique, à partir d'un échantillon de la pièce de viande, un test d'identification de la ou des empreintes génétiques du ou des individus dont la pièce de viande est issue, en ce qu'on compare la ou les empreintes génétiques à au moins une empreinte génétique obtenue à partir d'un échantillon, de préférence le cerveau ou la moelle épinière d'un animal, le cerveau ou la moelle épinière de cet animal ayant été soumis, après abattage, à un test de détection de protéines prion (PrPsc) spécifiques des encéphalopathies spongiformes transmissibles et en ce que, si les résultats de la comparaison révèlent 100 % d'homologie entre lesdites empreintes, on confirme de manière certaine l'absence de protéine prion (PrPsc) dans ladite pièce de viande.

L'invention a encore pour objet une installation pour la mise en oeuvre du procédé précité, caractérisée en ce qu'elle comprend au moins d'une part des moyens d'analyse pour la détermination d'empreintes génétiques et la détection de protéines prion spécifiques de l'encéphalopathie spongiforme transmissible, d'autre part des moyens informatiques pour le stockage et le traitement des résultats d'analyse.

L'invention sera bien comprise à la lecture de la description suivante d'exemples de réalisation.

Comme mentionné ci-dessus, le problème essentiel des encéphalopathies spongiformes transmissibles (EST) résulte du fait que ces maladies ne sont à ce jour détectables que sur des parties particulières de l'animal, en particulier le cerveau et la moelle épinière. Il est donc impossible à ce jour de confirmer, en l'absence d'éléments certains sur l'origine d'une pièce de viande, l'absence de contamination détectable de l'animal d'où est issue une telle pièce de viande. Le procédé, objet de l'invention, permet de résoudre ce problème technique. Ce procédé s'applique à tout produit à base de viande y compris des produits transformés.

Ce procédé consiste de manière générale à pratiquer, à partir d'un échantillon de la pièce de viande, un test d'identification de la ou des empreintes génétiques du ou des individus dont la pièce de viande est issue, à comparer cette empreinte génétique à au moins une empreinte génétique obtenue à partir d'un échantillon de cerveau ou de moelle épinière d'un animal, ce cerveau ou cette moelle épinière ayant été soumis, après abattage de l'animal, à un test de détection de protéines prion spécifiques des encéphalopathies spongiformes transmissibles et à confirmer, si les résultats de la comparaison révèlent 100 % d'homologie entre lesdites empreintes, l'absence de protéines prion PrPsc dans la pièce de viande.

Deux procédures peuvent être mises en oeuvre dans le cadre de ce procédé.

La première procédure consiste à soumettre après abattage un échantillon de cerveau ou de moelle épinière d'un animal abattu à un test de détection de protéines prion PrPsc spécifiques de la maladie et à un test de détermination de l'empreinte génétique puis à stocker dans une base de données l'empreinte génétique obtenue et à comparer ultérieurement, au moyen d'un logiciel de traitement approprié, l'empreinte génétique obtenue à partir de la pièce de viande à certifier aux empreintes génétiques mémorisées dans la base de données. La confirmation de l'absence de protéines prion dans la pièce de viande est obtenue si le résultat de la comparaison révèle 100 % d'homologie entre lesdites empreintes.

Dans la base de données, il sera possible d'associer si nécessaire, à chaque empreinte génétique obtenue sur un échantillon de cerveau ou de moelle épinière, les résultats du test de détection de protéines prion infectieuses effectué sur ce même échantillon. Toutefois, la législation actuelle obligeant les centres d'abattage à se débarrasser de carcasses d'animaux dont le test de détection de protéines prion infectieuses est positif, la base de données ne devrait contenir que des empreintes génétiques d'animaux testés négatifs. Si l'empreinte génétique n'est pas conforme à une empreinte génétique mémorisée, on ne pourra pas conclure car la pièce de viande pourrait être issue d'un animal infecté ou d'un animal qui n'a pas été testé par le procédé. A l'inverse, si l'empreinte génétique est conforme à une empreinte génétique mémorisée, on pourra conclure à l'absence de protéines prions spécifiques PrPsc. Cette première procédure consiste à effectuer des analyses systématiques sur tous les cerveaux ou moelles épinières d'animaux abattus et à procéder parallèlement à un test de détermination d'empreintes génétiques. Elle a l'avantage de pouvoir s'exempter de tout marquage de la pièce de viande notamment en ce qui concerne l'animal dont elle est issue. Cette procédure rend donc plus complexe les phases suivant la procédure d'abattage mais, à l'inverse, simplifie la traçabilité puisqu'elle peut s'en exempter totalement.

Suivant une autre procédure permettant la mise en oeuvre du procédé, on soumet, après abattage, un échantillon de cerveau ou de moelle épinière de l'animal abattu à un test de détection des protéines prion spécifiques de l'EST, on stocke ledit échantillon muni d'un code d'identification de l'animal, on identifie chaque pièce de viande issue de l'animal abattu par référence au code d'identification et, pour déterminer si une pièce de viande est bien issue de l'animal analysé et testé négatif dont elle porte le code d'identification, on pratique d'une part sur l'échantillon de cerveau ou de moelle épinière stocké, d'autre part sur la pièce de viande à certifier, un test de détermination de l'empreinte génétique et on vérifie l'homologie entre lesdites empreintes. Une absence d'homologie permettra d'affirmer de manière certaine que le marquage de la pièce de viande n'est pas conforme et qu'il y a donc peut être eu fraude. Cette seconde procédure oblige donc à mettre en oeuvre des opérations de traçabilité sur les pièces de viande commercialisées mais limite les procédures d'analyse après abattage. Le procédé, objet de l'invention, permet de garantir cette traçabilité.

Dans le cas de la mise en oeuvre de la première procédure, le contenu des bases de données pourra être accessible à tous ou uniquement réservé à des laboratoires spécialisés.

Dans les procédures décrites ci-dessus, on déterminera l'empreinte génétique généralement par séquençage ou par méthode RFLP (restriction fragment length polymorphism). Ces méthodes sont bien connues à ceux versés dans cet art et ne seront donc pas décrites plus en détail ci-après. La méthode RFLP nécessite de disposer simplement d'une machine PCR standard. Cette technique est aujourd'hui largement utilisée en routine. La technique du séquençage est une technique plus onéreuse mais qui peut toutefois être appliquée dans le cadre des procédés décrits ci-dessus.

Les tests de détection du prion pourront quant à eux être effectués au moyen de tests déjà commercialisés, en particulier par le CEA et Bio Rad ou par les Sociétés Prionics et Enfer Scientific. D'autres méthodes de détection du prion ont notamment été décrites dans la demande internationale PCT WO/98.16834.

La mise en oeuvre du procédé décrit ci-dessus peut être effectuée au moyen d'une installation relativement simple. Cette installation comprend au moins d'une part des moyens d'analyse pour la détermination d'empreintes génétiques et la détection de protéines prion spécifiques de l'encéphalopathie spongiforme transmissible, d'autre part des moyens informatiques pour le stockage et le traitement des résultats d'analyse.

## Revendications

1. Procédé pour confirmer l'absence de protéines prion (PrPsc) infectieuses spécifiques d'encéphalopathies spongiformes transmissibles (EST), telles que l'encéphalopathie spongiforme bovine ou la maladie de scrapie ou le chronic wasting disease, dans des pièces de viande, autres que le cerveau ou la moelle épinière, issues notamment de bovins, de moutons ou de gibier, et en cours de commercialisation,
**caractérisé en ce qu'**on pratique, à partir d'un échantillon de la pièce de viande, un test d'identification de la ou des empreintes génétiques du ou des individus dont la pièce de viande est issue, **en ce qu'**on compare la ou les empreintes génétiques à au moins une empreinte génétique obtenue à partir d'un échantillon, de préférence le cerveau ou la moelle épinière d'un animal, le cerveau ou la moelle. épinière de cet animal ayant été soumis, après abattage, à un test de détection de protéines prion (PrPsc) spécifiques des encéphalopathies spongiformes transmissibles et **en ce que**, si les résultats de la comparaison révèlent 100 % d'homologie entre lesdites empreintes, on confirme de manière certaine l'absence de protéine prion (PrPsc) dans ladite pièce de viande.

2. Procédé selon la revendication 1,
**caractérisé en ce qu'**on soumet, après abattage, un échantillon de cerveau ou de moelle épinière de l'animal abattu à un test de détection de protéines prion (PrPsc) spécifiques de la maladie et à un test de détermination de l'empreinte génétique, **en ce qu'**on stocke dans une base de données l'empreinte génétique obtenue, **en ce qu'**on associe si nécessaire à chaque empreinte le résultat du test de détection de prion, **en ce qu'**on compare, au moyen d'un logiciel de traitement approprié, l'empreinte génétique obtenue à partir de la pièce de viande à certifier aux empreintes génétiques mémorisées, **en ce qu'**on confirme, si le résultat de la comparaison révèle 100 % d'homologie entre lesdites empreintes, l'absence de protéines prion (PrPsc) spécifiques dans la pièce de viande.

3. Procédé selon la revendication 1,
**caractérisé en ce qu'**on soumet, après abattage, un échantillon de cerveau ou de moelle épinière de l'animal abattu à un test de détection des protéines prion spécifiques de l'EST, on stocke ledit échantillon muni d'un code d'identification, **en ce qu'**on identifie chaque pièce de viande issue de l'animal abattu par référence au code d'identification de l'animal et **en ce que**, pour vérifier si une pièce de viande est bien issue de l'animal testé négatif dont elle porte le code d'identification, on pratique d'une part sur l'échantillon de cerveau ou de moelle épinière stocké, d'autre part sur la pièce de viande à certifier, un test de détermination de l'empreinte génétique, et on vérifie l'homologie entre lesdites empreintes.

4. Procédé selon l'une des revendications 1 à 3,
**caractérisé en ce qu'**on détermine l'empreinte génétique par séquençage ou par méthode RFLP (restriction fragment length polymorphism).

5. Installation pour la mise en oeuvre du procédé selon l'une des revendications 1 à 4,
**caractérisée en ce qu'**elle comprend au moins d'une part des moyens d'analyse pour la détermination d'empreintes génétiques et la détection de protéines prion spécifiques de l'encéphalopathie spongiforme transmissible, d'autre part des moyens informatiques pour le stockage et le traitement des résultats d'analyse.

## Claims

1. Method for confirming the absence of specific infectious prion proteins (PrPsc) of transmissible spongiform encephalopathies (TSE), such as bovine spongiform encephalopathy or the disease of scrapie or the chronic wasting disease, in pieces of meat other than the brain or the spinal cord, originating more especially from cows, sheep or game, and in the process of being marketed, **characterised in that**, from a sample of the piece of meat, a test is carried out to identify the genetic fingerprint or fingerprints of the individual or individuals from which the piece of meat originates, **in that** the genetic fingerprint or fingerprints is or are compared with at least one genetic fingerprint obtained from a sample, preferably the brain or the spinal cord of an animal, the brain or the spinal cord of this animal having been subjected, after slaughtering, to a test for detecting specific prion proteins (PrPsc) of transmissible spongiform encephalopathies, and **in that**, if the results of the comparison reveal 100 % homology between said fingerprints, the absence of prion proteins (PrPsc) in said piece of meat is confirmed with certainty.

2. Method according to claim 1, **characterised in that**, after slaughtering, a sample of brain or spinal cord from the slaughtered animal is subjected to a test for detecting specific prion proteins (PrPsc) of the disease and to a test for determining the genetic fingerprint, **in that** the genetic fingerprint obtained is stored in a database, **in that**, if necessary, the result of the prion detection test is associated with each fingerprint, **in that** the genetic fingerprint obtained from the piece of meat to be certified is compared, by means of appropriate processing software, with the memorised genetic fingerprints, and **in that**, if the result of the comparison reveals 100 % homology, the absence of specific prion proteins (PrPsc) in the piece of meat is confirmed.

3. Method according to claim 1, **characterised in that**, after slaughtering, a sample of brain or spinal cord from the slaughtered animal is subjected to a test for detecting specific prion proteins of TSE, said sample, provided with an identification code, is stored, **in that** each piece of meat originating from the slaughtered animal is identified by reference to the identification code of the animal, and **in that**, to verify whether a piece of meat has indeed originated from the animal which tested negative, and of which it bears the identification code, a test for determining the genetic fingerprint is carried out on the stored sample of brain or spinal cord, on the one hand, and on the piece of meat to be certified, on the other hand, and the homology between said fingerprints is verified.

4. Method according to one of claims 1 to 3, **characterised in that** the genetic fingerprint is determined by sequencing or by the RFLP (restriction fragment length polymorphism) method.

5. Installation for carrying out the method according to one of claims 1 to 4, **characterised in that** it comprises analysing means for determining genetic fingerprints and for detecting specific prion proteins of transmissible spongiform encephalopathy, at least on the one hand, and computer means for storing and processing the analysis results, on the other hand.

## Patentansprüche

1. Verfahren zur Bestätigung des Nichtvorhandenseins von infektiösen, für die übertragbaren spongiformen Enzephalopathien (TSE) wie beispielsweise die bovine spongiforme Enzephalopathie oder Scrapie oder die Chronic Wasting Disease spezifischen Prion-Proteinen (PrPsc) in anderen sich im Handel befindlichen Fleischstücken als dem Gehirn oder dem Rückenmark, insbesondere von Rindern, Schafen oder Wild, **dadurch gekennzeichnet, dass** bei einer Probe des Fleischteils ein Identifizierungstest des oder der genetischen Fingerabdrücke des oder der Individuen, von dem oder denen das Fleischstück stammt, durchgeführt wird, dass der oder die genetischen Abdrücke mit mindestens einem genetischen Abdruck verglichen wird, der von einer Probe, vorzugsweise vom Gehirn oder dem Rückenmark eines Tieres stammt, wobei das Gehirn oder das Rückenmark dieses Tieres nach der Schlachtung einem Test zum Nachweis von Prion-Proteinen (PrPsc), die für übertragbare spongiforme Enzephalopathien spezifisch sind, unterzogen wurde, und dass das Nichtvorhandensein von Prion-Proteinen (PrPsc) in dem genannten Fleischstück mit Sicherheit bestätigt wird, wenn sich die Vergleichsergebnisse der genannten Abdrücke als 100%ig übereinstimmend erweisen.

2. Verfahren nach Anspruch 1, **dadurch gekennzeichnet, dass** nach der Schlachtung eine Probe des Gehirns oder des Rückenmarks des geschlachteten Tieres einem Test zum Nachweis von Prion-Proteinen (PrPsc), die spezifisch für die Krankheit sind, und einem Test zur Bestimmung des genetischen Fingerabdrucks unterzogen wird, dass der erhaltene genetische Abdruck in einer Datenbank gespeichert wird, dass, wenn notwendig, jedem Abdruck das Ergebnis des Tests zum Nachweis von Prionen beigefügt wird, dass mit Hilfe einer geeigneten Verarbeitungssoftware der von dem zu bestätigenden Fleischstück erhaltene genetische Abdruck mit den gespeicherten genetischen Abdrücken zu vergleichen ist, dass das Nichtvorhandensein von spezifischen Prion-Proteinen (PrPsc) in dem Fleischstück bestätigt wird, wenn sich das Vergleichsergebnis der genannten Abdrücke als 100%ig übereinstimmend erweist.

3. Verfahren nach Anspruch 1, **dadurch gekennzeichnet, dass** nach der Schlachtung eine Gehirn- oder Rückenmarksprobe des geschlachteten Tieres einem Test zum Nachweis von spezifischen Prion-Proteinen für TSE unterzogen wird und die mit einem Identifikationscode versehene genannte Probe aufbewahrt wird, dass jedes von dem geschlachteten Tier stammende Fleischstück entsprechend des Identifikationscodes des Tieres identifiziert wird und dass zur Kontrolle, ob ein Fleischstück wirklich von dem als negativ getesteten Tier, dessen Identifikationscode es aufweist, stammt, einerseits bei der aufbewahrten Gehirn- oder Rückenmarksprobe und andererseits bei dem zu bestätigenden Fleischstück ein Test zum Nachweis des genetischen Fingerabdrucks durchgeführt wird, und die Übereinstimmung der genannten Abdrücke geprüft wird.

4. Verfahren nach einem der Ansprüche 1 bis 3, **dadurch gekennzeichnet, dass** die Bestimmung des genetischen Fingerabdrucks durch Sequenzierung oder durch das RFLP-Verfahren (restriction fragment length polymorphism) erfolgt.

5. Vorrichtung für die Durchführung des Verfahrens nach einem der Ansprüche 1 bis 4, **dadurch gekennzeichnet, dass** sie einerseits mindestens die Analysemittel zur Bestimmung von genetischen Fingerabdrücken und den Nachweis von spezifischen Prion-Proteinen der übertragbaren spongiformen Enzephalopathie und andererseits die Datenverarbeitungsmittel für die Speicherung und Verarbeitung der Analyseergebnisse umfasst.
